(19) Europäisches Patentamt
European Patent Office
Office européen des brevets

(11) **EP 3 062 352 A1**

(12) **DEMANDE DE BREVET EUROPEEN**

(43) Date de publication:
**31.08.2016 Bulletin 2016/35**

(51) Int Cl.:
*H01L 31/0304* (2006.01)    *H01L 31/0352* (2006.01)
*H01L 27/146* (2006.01)    *H01L 31/105* (2006.01)
*A61F 2/14* (2006.01)    *A61N 1/05* (2006.01)
*H01S 5/50* (2006.01)

(21) Numéro de dépôt: **15305288.1**

(22) Date de dépôt: **25.02.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(71) Demandeur: **ALCATEL LUCENT**
**92100 Boulogne-Billancourt (FR)**

(72) Inventeurs:
• **Garreau, Alexandre**
  **91460 Marcoussis (FR)**

• **Aubry, Raphaël**
  **91460 Marcoussis (FR)**
• **Brenot, Romain**
  **91767 Palaiseau (FR)**

(74) Mandataire: **Berthier, Karine et al**
**Alcatel-Lucent International**
**148/152, route de la Reine**
**92100 Boulogne-Billancourt (FR)**

(54) **Dispositif optoélectronique**

(57)    Le dispositif optoélectronique comporte une matrice incluant au moins un composant optoélectronique comprenant une couche active de nitrure de gallium GaN comportant de multiples puits quantiques InGaN/GaAsN ou InGaN/AlGaN sur un substrat de nitrure de gallium GaN dopé p et recouverte d'une couche de nitrure de gallium GaN dopé n. Le substrat de nitrure de gallium GaN dopé p forme une colonne de p-GaN recouverte d'une couche isolante en matériau biocompatible.

Le dispositif peut comporter une matrice comprenant une pluralité de composants électroniques qui peuvent être de hauteurs différentes. Le composant optoélectronique peut être une photodiode ou un amplificateur optique semiconducteur à cavité verticale. Ce dispositif optoélectronique est utilisable dans des prothèses épirétiniennes ou sous-rétiniennes. Une même prothèse épirétinienne ou sous-rétinienne peut comprendre une matrice de photodiodes et une matrice d'amplificateurs optiques semiconducteurs SOA à cavité verticale.

**FIG. 3**

(c)

33
34
31
30    p GaN
n GaN
32

EP 3 062 352 A1

**Description**

La présente invention se rapporte au domaine des dispositifs optoélectroniques implantables, utilisables en particulier dans les prothèses rétiniennes destinée à compenser la dégradation des cellules photoréceptrices de l'oeil humain.

ARRIERE PLAN

L'oeil humain, ou globe oculaire, est une structure creuse de forme globalement sphérique. La couche la plus interne de la partie postérieure de l'oeil est la rétine. La rétine est une structure nerveuse, composée de nombreux photorécepteurs et de neurones traitant et acheminant l'information visuelle vers le cerveau par le nerf optique. Au point de sortie du nerf optique, la rétine est interrompue : c'est la tache aveugle, à proximité de laquelle se trouve la tache jaune, ou macula, comportant une fossette centrale : la fovéa.

Des cellules nerveuses photoréceptrices spécialisées tapissent la paroi interne du fond de l'oeil, les cônes et les bâtonnets nommés ainsi en raison de leur forme qui contiennent des substances photosensibles. Les bâtonnets, sensibles à l'intensité lumineuse, sont des photorécepteurs plus particulièrement destinés à la vision crépusculaire et les cônes, responsables de la vision des couleurs, sont plus particulièrement destinés à la vision diurne. Les cônes se subdivisent en trois familles de cellules, chacune ayant son pic de sensibilité dans une zone bien déterminée du spectre (bleu-violet, vert, et jaune-vert).

La dégradation des cellules photoréceptrices de l'oeil humain peut être due par exemple à la dégénérescence maculaire liée à l'âge (DMLA) ou à la rétinite pigmentaire d'origine génétique. Les photorécepteurs (cônes et bâtonnets) sont les cellules de la rétine sensibles à la lumière, tandis que les autres neurones de traitement des signaux captés par les photorécepteurs envoient des informations au cerveau via le nerf optique. Lorsque les cellules photoréceptrices dégénèrent, la rétine ne peut plus répondre à la lumière. Cependant, les autres neurones demeurent en nombre suffisant pour que leur stimulation électrique entraîne la perception de la lumière par le cerveau.

Afin de remédier aux déficiences des cellules photoréceptrices, deux voies ont été explorées : l'implantation de prothèses rétiniennes, et l'approche optogénétique.

La technique optogénétique consiste à modifier des neurones pour les rendre sensibles à la lumière, en incorporant une protéine sensible à la lumière dans la membrane cellulaire. En rendant chaque cellule sensible à la lumière, la vision peut potentiellement être restaurée à une acuité proche de la normale. Cependant, la vision artificielle basée sur l'approche optogénétique présente un inconvénient majeur. Les cellules modifiées exigent une lumière bleue (460 nm) et très brillante pour être activées, soit une intensité lumineuse nécessaire qui est environ sept fois plus importante que le seuil de sensibilité à la lumière observés normalement chez les personnes en bonne santé.

Les prothèses rétiniennes comporte un dispositif optoélectronique incluant une matrice de composants optoélectroniques qui sont activés, soit par de la lumière entrant dans l'oeil dans le cas de la prothèse dite « sous-rétinienne », soit par un signal électrique provenant d'une microcaméra montée à l'extérieur de l'oeil, dans le cas de la prothèse dite « épirétinienne ». Les différents types d'implants utilisés font appel à une technologie basée sur le silicium qui est facile à mettre en oeuvre et permet l'élaboration de dispositifs nanométriques. Toutefois le silicium est un matériau opaque dans le domaine optique visible.

La solution épirétinienne consiste à placer un implant électronique en avant de la rétine pour stimuler les neurones. L'implant épirétinien lui-même n'est pas sensible à la lumière et doit être relié à une microcaméra disposée à l'extérieur de l'oeil. L'implant épirétinien nécessite un « codeur » dont la fonction est de remplir le rôle des neurones de la couche interne de la rétine qui effectuent un traitement préliminaire de l'information visuelle.

Aux Etats-Unis, la « Food and Drug Administration » (FDA) a autorisé en février 2013 l'utilisation de la première prothèse épirétinienne destinée à traiter les patients atteints de rétinite pigmentaire évoluée. Cette prothèse épirétinienne appelée « Argus II Retinal Prosthesis System » est fabriquée par la société « Second Sight Medical Products Inc. ». Cette prothèse épirétinienne constitue une référence avec une matrice de soixante électrodes, et a déjà été testée sur des patients dans le monde entier.

La prothèse sous-rétinienne est placée sous la rétine pour remplacer les cellules nerveuses photoréceptrices détruites, ce qui est chirurgicalement plus difficile à réaliser mais permet de stimuler les neurones dans une position plus naturelle. La prothèse sous-rétinienne convertit la lumière incidente en un signal électrique qui est transmis aux neurones (cellules bipolaires). La prothèse sous-rétinienne est elle-même sensible à la lumière et n'a pas besoin d'un appareillage externe La prothèse sous-rétinienne de référence « Retina » est fabriquée par la société « Retina Implant AG ».

On considère que pour la lecture d'un texte, pour avoir une locomotion autonome ou reconnaître un visage, la résolution minimale doit être de plus de 1000 pixels. La prothèse sous-rétinienne est sensible à la lumière avec une matrice de 1500 pixels. Dans le cas de la prothèse épirétinienne, la résolution n'est que de 60 pixels. Cependant des tests cliniques réalisés avec les deux types de prothèses donnent des résultats équivalents, alors qu'il y a un nombre d'électrodes sensiblement plus important dans une prothèse sous-rétinienne. Lorsque le nombre de composants optoélectroniques augmente, les matrices deviennent plus denses avec des composants optoélectroniques plus petits. Un positionnement plus précis des composants optoélectroniques individuels devient alors indispensable pour augmenter la proximité entre le composant optoélectronique et la couche de cellules ganglionnaires. Cela permet à chaque composant optoélectronique d'activer une petite portion de la rétine afin d'augmenter l'acuité visuelle.

Par ailleurs les prothèses rétiniennes connues sont des dispositifs plans en deux dimensions (2D) qui ne sont pas capable de faire une stimulation en trois dimensions (3D), ce qui constitue une limitation importante de leurs performances.

RESUME

Des solutions plus performantes que les solutions actuelles, et ne présentant pas les inconvénients précités, sont donc attendues.

La solution proposée est un dispositif optoélectronique comportant une matrice incluant au moins un composant optoélectronique comprenant une couche active de nitrure de gallium GaN comportant de multiples puits quantiques InGaN/GaAsN (nitrure d'indium-gallium / nitrure d'arsenic-gallium) ou InGaN/AlGaN (nitrure d'indium-gallium / nitrure d'aluminium-gallium) sur un substrat de nitrure de gallium GaN dopé p et recouverte d'une couche de nitrure de gallium GaN dopé n.

Le matériau semiconducteur GaN présente l'intérêt d'avoir une bonne stabilité chimique et d'être biocompatible. Pour cette raison, il est possible d'encapsuler des matières mal reçues par l'organisme humain dans ce matériau qui crée une barrière de protection.

Le matériau semiconducteur GaN a aussi la caractéristique d'être transparent dans le domaine de longueur d'onde de la lumière visible. De cette manière, les cellules rétiniennes encore fonctionnelles ne sont pas gênées par l'opacité de la prothèse rétinienne. En outre les cellules rétiniennes non atteintes sont encore stimulées parce qu'elles ne sont pas masquées par la prothèse rétinienne vis-à-vis de la lumière visible pénétrant dans l'oeil.

Ainsi les dispositifs optoélectroniques comportant une matrice comprenant des composants optoélectroniques basés sur une structure de nitrure de gallium GaN comportant des multiple puits quantiques MQW (Multi-Quantum Wells) InGaN/GaAsN ou InGaN/AlGaN présentent l'avantage de laisser passer la lumière entre deux composants optoélectroniques voisins.

Selon un aspect, le substrat de nitrure de gallium GaN dopé p forme une colonne de p-GaN.

Selon un autre aspect, la colonne de p-GaN est recouverte d'une couche isolante en matériau biocompatible choisi parmi le carbone, le diamant, le dioxyde de titane, la silice, le nitrure de silicium ou le nitrure de gallium.

Selon encore un autre aspect, le rapport entre la hauteur et la dimension transversale de la colonne de p-GaN est inférieur à 20.

Selon un mode de réalisation, le composant optoélectronique est une photodiode. L'utilisation d'une matrice transparente de photodiodes comportant de multiple puits quantiques InGaN/GaAsN ou InGaN/AlGaN permet de se passer de la microcaméra utilisée aujourd'hui dans le cas d'une prothèse épirétinienne.

Selon un autre mode de réalisation, le composant optoélectronique est un amplificateur optique semiconducteur SOA à cavité verticale. Avantageusement deux réflecteurs de Bragg distribués sont disposés respectivement de part et d'autre de la couche active GaN comportant de multiples puits quantiques de manière à définir une cavité optique.

Une matrice transparente d'amplificateurs optiques semiconducteurs SOA permet d'amplifier la lumière bleue, jaune ou verte pour améliorer les résultats obtenus avec la technique optogénétique, ou avec des prothèses épirétiniennes ou sous-rétiniennes.

Selon un autre mode de réalisation, le dispositif optoélectronique comprend au moins une photodiode et au moins un amplificateur optique semiconducteur SOA à cavité verticale.

Selon encore un autre mode de réalisation, le dispositif optoélectronique comporte une matrice comprenant une pluralité de composants optoélectroniques. Avantageusement les composants optoélectroniques ont des hauteurs différentes.

Les photodiodes et les amplificateurs optiques semiconducteurs SOA présentent des hauteurs différentes afin de stimuler plus précisément la couche de cellules ganglionnaires CCG et/ou le nerf optique.

De préférence les composants optoélectroniques sont espacés d'une distance E telle que $E = \pi (350/2)^2 \times 1/n$ où n est le nombre de composants optoélectroniques de la matrice.

Selon un autre aspect, la matrice de composants optoélectroniques est une matrice en trois dimensions 3D de photodiodes et/ou d'amplificateurs optiques semiconducteurs SOA à cavité verticale.

On propose aussi une prothèse épirétinienne comprenant un tel dispositif optoélectronique.

On propose encore une prothèse sous-rétinienne comprenant un tel dispositif optoélectronique.

Selon un aspect, une prothèse épirétinienne ou sous-rétinienne comprend une matrice de photodiodes.

Selon un autre aspect, une prothèse épirétinienne ou sous-rétinienne comprend une matrice d'amplificateurs optiques semiconducteurs à cavité verticale.

Selon encore un autre aspect, on peut intégrer simultanément dans une même prothèse épirétinienne ou sous-rétinienne, au moins une matrice de photodiodes et au moins une matrice d'amplificateurs optiques semiconducteurs SOA à cavité verticale afin de stimuler les neurones à la fois par injection d'un signal électrique et par amplification de la lumière bleue, verte ou jaune.

BREVE DESCRIPTION

D'autres caractéristiques et avantages apparaîtront au cours des exemples suivants de réalisation particuliers, donnés bien entendu à titre illustratif et non limitatif, et dans le dessin annexé sur lequel la figure unique représente

D'autres caractéristiques et avantages apparaîtront à la lecture de la description qui suit d'un mode de réalisation, donné bien entendu à titre illustratif et non limitatif, et dans le dessin annexé sur lequel

- la figure 1 illustre une vue en coupe schématique d'un oeil humain,
- la figure 2 illustre une vue en coupe schématique de la rétine,
- les figures 3a, 3b et 3c illustrent schématiquement un composant optoélectronique selon l'invention,
- la figure 4 illustre schématiquement un mode de réalisation d'un dispositif optoélectronique comprenant une photodiode applicable à une prothèse sous-rétinienne,
- la figure 5 illustre schématiquement un mode de réalisation d'un dispositif optoélectronique comprenant une photodiode applicable à une prothèse épirétinienne,
- la figure 6 illustre schématiquement un mode de réalisation d'un dispositif optoélectronique comprenant un amplificateur optique semiconducteur applicable à une prothèse sous-rétinienne,
- la figure 7 illustre schématiquement un mode de réalisation d'un dispositif optoélectronique comprenant un amplificateur optique semiconducteur applicable à une prothèse épirétinienne,
- la figure 8 illustre un mode de réalisation d'un dispositif optoélectronique comprenant une photodiode et un amplificateur optique semiconducteur applicable à une prothèse sous-rétinienne.
- la figure 9 illustre un mode de réalisation d'un dispositif optoélectronique comprenant une photodiode et un amplificateur optique semiconducteur applicable à une prothèse épirétinienne,
- la figure 10 illustre schématiquement un mode de réalisation d'une matrice de composants optoélectroniques.

La terminologie directionnelle comme « gauche et « droite », « haut et « bas », « avant » et « arrière », « vertical » et « horizontal », « supérieur » et « inférieur », etc... est utilisée ici en référence à l'orientation des figures décrites. Parce que les éléments composant les modes de réalisation peuvent être placés dans des orientations différentes, la terminologie directionnelle n'est utilisée ici qu'à des fins d'illustration et n'est nullement limitative.

DESCRIPTION DETAILLEE

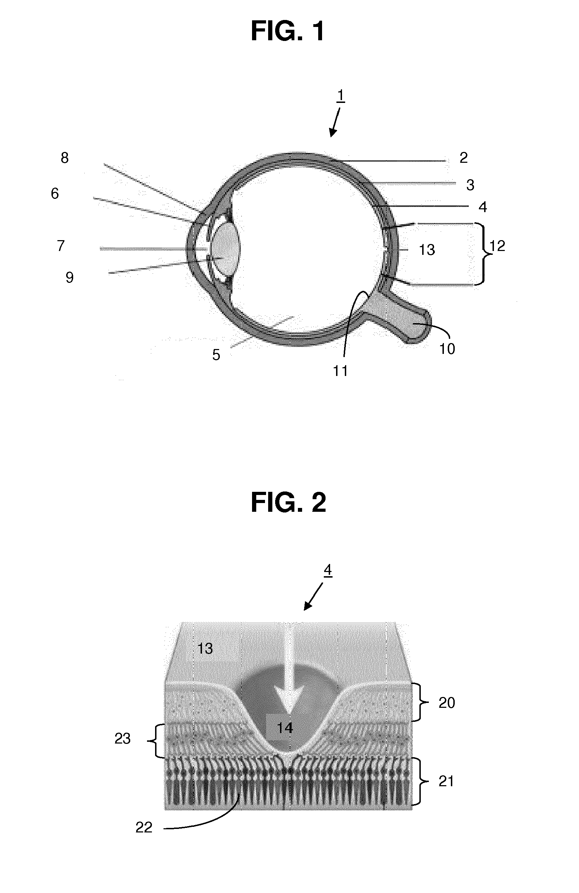

La figure 1 illustre schématiquement en coupe un oeil humain **1.** Il est formé de trois membranes superposées **2, 3, 4** entourant une substance gélatineuse appelée le corps vitré **5.**
La partie antérieure de l'oeil, recevant la lumière comprend

- l'iris **6** percé en son centre par une ouverture circulaire : la pupille **7,** qui laisse passer la lumière et dont l'ouverture s'adapte automatiquement à l'intensité lumineuse perçue,
- la cornée **8** qui est une membrane transparente circulaire et bombée qui permet le passage des rayons lumineux,
- le cristallin **9** qui permet de focaliser l'image sur la rétine en fonction de sa distance.

La rétine **4** est la membrane qui tapisse la face interne du fond postérieur de l'oeil. Les cellules nerveuses de la rétine transforment l'énergie lumineuse en signaux électriques qui sont transmis au cerveau par le nerf optique **10.** La tache aveugle **11** est la zone où les fibres se réunissent pour former le nerf optique, qui ne renferme aucune cellule photosensible. A proximité, la macula **12,** ou tache jaune, est formée de nombreuses cellules visuelles.
La zone la plus sensible de la rétine, dépourvue de capillaires sanguins, est appelée la fovéa **13.** La fovéa **13** est une petite zone de la rétine située dans la macula **12** (environ 6 mm de diamètre) qui est sensible aux couleurs et sert pour la précision de la vision. Le fovéola **14** (environ 0,35 mm de diamètre) se trouve dans le centre de la fovéa **13** (environ 1,5 mm de diamètre) et contient uniquement des cônes. La fovéa **13** est le point de la rétine **4** ayant la meilleure acuité visuelle : c'est là que les rayons lumineux arrivent directement, avec le moins d'interférences, et c'est là que la densité de cellules photoréceptrices est la plus importante. Dans le fovéola **14,** les cônes photorécepteurs sont plus longs, plus minces et plus densément disposés que partout ailleurs dans la rétine **4.** Ceci permet au fovéola **14** d'avoir l'acuité visuelle la plus élevée de la rétine **4.** Les cellules photoréceptrices transforment l'énergie lumineuse en impulsion nerveuse transmise au nerf optique.
Comme illustré sur la coupe schématique de la figure 2, la rétine **4** est composée d'un empilement de plusieurs couches dans le sens radial. La couche externe **20,** ou couche de cellules ganglionnaires CCG, empêche la lumière de diffuser dans l'oeil. La couche interne **21,** ou couche des photorécepteurs PR, est formée de cellules nerveuses réceptrices spécialisées **22,** les cônes et les bâtonnets détectant la lumière et les neurones qui traitent et acheminent l'information visuelle vers le cerveau. La couche intermédiaire **23,** ou couche nucléaire interne CNI, contient des cellules de liaison comme les cellules bipolaires.

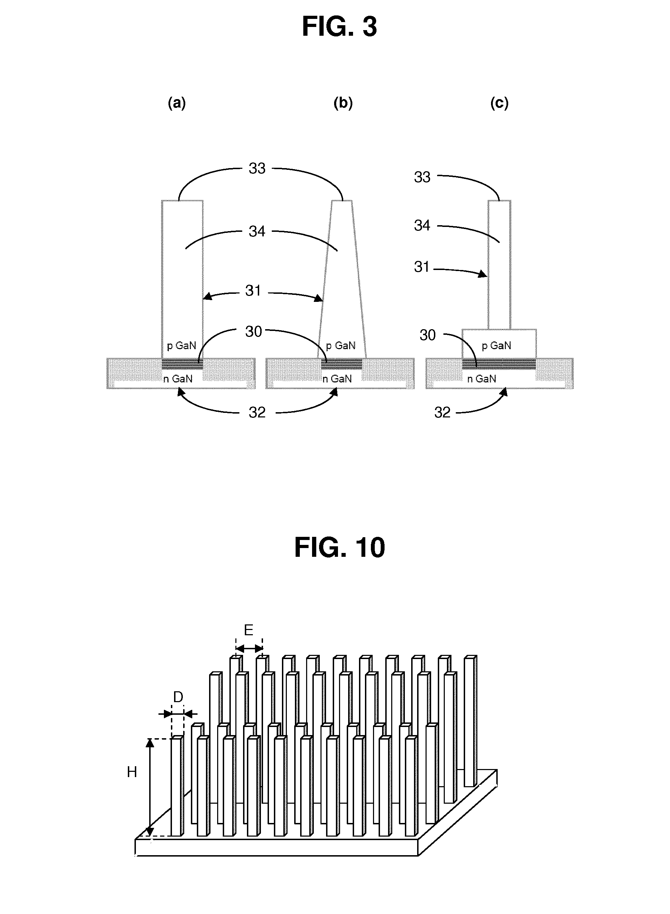

Il existe plusieurs types de prothèses rétiniennes qui reposent sur un dispositif optoélectronique comportant des composants optoélectroniques basés sur un concept commun illustré schématiquement par les figures 3a-3c. Ce concept repose sur la réalisation par une ou plusieurs épitaxies d'une couche active GaN **30** intrinsèque à multiple puits quantiques InGaN/GaAsN (nitrure d'indium-gallium / nitrure d'arsenic-gallium) ou InGaN/AlGaN (nitrure d'indium-gallium / nitrure d'aluminium-gallium) sur un substrat **31** de nitrure de gallium GaN dopé p. On entend par matériau intrinsèque un matériau semiconducteur sans dopage et/ou sans impureté. On rappelle que l'épitaxie est une croissance cristalline d'un matériau, réalisée en général sur le même matériau, avec respect de la maille et des orientations cristallines. Au sommet de chaque couche active GaN **30,** une couche de nitrure de gallium GaN dopée n **32** est réalisée pour terminer l'épitaxie.

Le procédé appliqué sur la face arrière **33** du substrat p-GaN **31,** s'appuyant sur l'amincissement et le polissage du substrat p-GaN **31** jusqu'au niveau de hauteur souhaité, permet d'obtenir des colonnes de p-GaN **34.** Les colonnes de p-GaN **33** sont obtenues par gravure sélective de la couche de substrat p-GaN **31,** par exemple par plasma de chlorure ICP.

La colonne p-GaN **34** doit être suffisamment longue pour pouvoir stimuler les cellules de la rétine. Le rapport entre la hauteur et la dimension transversale (largeur ou diamètre) de la colonne p-GaN 34 est de préférence inférieur à 20, afin d'éviter une rupture de la colonne. Les colonnes p-GaN **34** peuvent avoir des hauteurs différentes, afin de pouvoir stimuler différentes couches de la rétine. Les différentes hauteurs sont réalisées par exemple par gravure sélective du substrat p-GaN **31** en procédant à partir de la face arrière. La colonne p-GaN **34** peut avoir la forme globale d'une tige à bords parallèles (figure 3a), d'une pyramide tronquée (figure 3b) ou d'une tige mince surmontant une base plus large (figure 3c). Dans la suite de la description, on considère une tige mince à bords parallèles surmontant une base plus large comme illustré sur la figure 3c.

Plusieurs composants optoélectroniques comportant une telle structure à multiples puits quantiques peuvent être réalisés en utilisant la technologie de croissance de zone sélective SAG (Selective Area Growth) afin de pouvoir amplifier ou détecter plusieurs longueurs d'onde (bleu, vert, jaune). Les différents composants optoélectroniques disposés sur une même matrice sont électriquement séparés par une zone de GaN implanté ou une zone de GaN semi-isolant, de cette façon l'isolation entre les composants optoélectroniques est réalisée, et la matrice reste transparente.

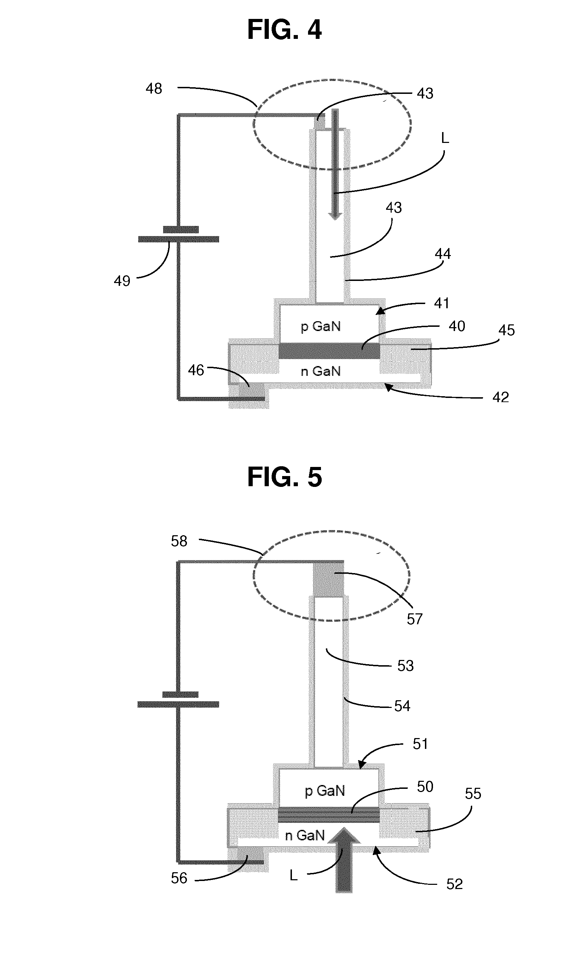

La figure 4 illustre schématiquement un mode de réalisation d'un dispositif optoélectronique, comprenant au moins une photodiode à base de GaN, destiné à être utilisé dans une prothèse sous-rétinienne.

Une couche active GaN **40** absorbante à multiple puits quantiques InGaN/GaAsN ou InGaN/AlGaN est réalisée par épitaxie sur un substrat **41** de nitrure de gallium GaN dopé p. Une couche **42** de nitrure de gallium GaN dopée n est déposée au-dessus de la couche absorbante GaN **40.** Par amincissement et polissage du substrat p-GaN **41,** on dégage une colonne **de p-GaN 43** jusqu'au niveau de hauteur souhaité. La colonne de p-GaN **43** est recouverte d'une couche isolante **44** en matériau diélectrique ou en matériau semi-isolant. En outre, le matériau constitutif de la couche isolante **44** doit offrir une bonne biocompatibilité comme le carbone, le diamant, le dioxyde de titane, les matériaux diélectriques communs (silice, nitrure de silicium,...) ou le matériau semi-isolant GaN. L'isolation est complétée par l'implantation de GaN semi-isolant **45** pour séparer les composants optoélectroniques les uns des autres, afin de permettre de polariser de manière indépendante chacun des composants optoélectroniques d'une matrice.

Sur la face avant de la couche de nitrure de gallium GaN dopée n **42** est disposé un contact métallique **46.** Le contact métallique **46** sur la face avant de la couche n-GaN **42** permet de polariser la prothèse rétinienne. Un autre contact métallique **47** est placé à l'extrémité supérieure de la colonne de p-GaN **43** correspondant à la zone de la rétine **48** qui est stimulée. Les contacts métalliques **46** et **47** sont reliés par un générateur électrochimique **49** (pile ou accumulateur) qui impose une tension entre eux. Parce que la lumière **L** doit traverser la colonne p-GaN **43** pour atteindre la couche active GaN **40** absorbante, le contact métallique **47** ne doit pas couvrir la totalité de la surface supérieure de la colonne p-GaN **43.** Un photocourant apparaît qui va stimuler les différentes couches de la rétine.

Dans le mode de réalisation illustré schématiquement sur la figure 5, un dispositif optoélectronique comprenant au moins une photodiode à base de GaN est destiné à être utilisé dans une prothèse épirétinienne est illustré.

Une couche active GaN **50** absorbante à multiple puits quantiques InGaN/GaAsN ou InGaN/AlGaN est réalisée par épitaxie sur un substrat **51** de nitrure de gallium GaN dopé p. Une couche **52** de nitrure de gallium GaN dopée n est déposée au-dessus de la couche absorbante GaN **50.** Par amincissement et polissage du substrat p-GaN **51,** on dégage une colonne de p-GaN **53** jusqu'au niveau de hauteur souhaité. La colonne de p-GaN **53** est recouverte d'une couche isolante **54** en matériau diélectrique ou en matériau semi-isolant. L'isolation est complétée par l'implantation de GaN semi-isolant **55.** Chaque photodiode d'une matrice peut ainsi être polarisée de façon indépendante de sa voisine en fonction du besoin médical. Un contact métallique **56** est placé sur la face avant de la couche n-GaN **52** et un autre contact métallique **57** est placé à l'extrémité de la colonne de p-GaN **53** correspondant à la zone de la rétine **58** à stimuler. Parce que la lumière **L** doit traverser la couche n-GaN **52** pour atteindre la couche absorbante GaN **50,** le contact métallique **56** ne doit pas couvrir la totalité de la surface de la face avant de la couche n-GaN **52.** Un photocourant apparaît qui va stimuler les différentes couches de la rétine. Toutefois le contact métallique **57** peut couvrir la totalité de la surface de l'extrémité de la colonne p-GaN **53** car le photocourant induit suffit pour stimuler les différentes couches de la rétine. En effet il n'y a pas d'intérêt à transmettre de la lumière en dehors de la zone épirétinienne où il n'y a pas de cellules photoréceptrices.

Le remplacement de la rétine par des matrices contenant des milliers, voire des millions, de composants optoélectroniques à base de semiconducteurs, comme ces photodiodes, vont permettre de transformer la lumière en signal électrique, qui sera ensuite transmis aux fibres visuelles encore fonctionnelles.

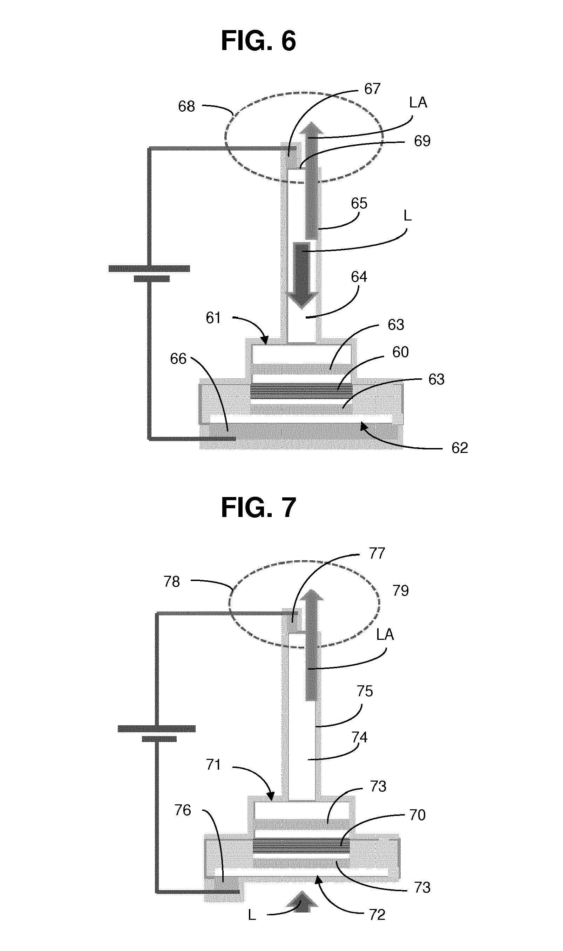

On considérera maintenant la figure 6 qui illustre schématiquement un mode de réalisation d'un dispositif optoélectronique, comprenant au moins un amplificateur optique semiconducteur à base de GaN à cavité verticale, destiné à être utilisé dans une prothèse sous-rétinienne.

Une couche active GaN **60** amplificatrice à multiple puits quantiques InGaN/GaAsN ou InGaN/AlGaN est réalisée par épitaxie sur un substrat **61** de nitrure de gallium GaN dopé p. Une couche **62** de nitrure de gallium GaN dopée n est déposée au-dessus de la couche amplificatrice GaN **60**. Deux réflecteurs de Bragg distribués DBR **63** (pour « Distributed Bragg Reflector » en anglais) sont disposés respectivement de part et d'autre de la couche amplificatrice GaN **60**.

Par amincissement et polissage du substrat p-GaN **61**, on dégage une colonne de p-GaN **64** jusqu'au niveau de hauteur souhaité. La colonne de p-GaN **64** est recouverte d'une couche isolante **65** en matériau diélectrique ou en matériau semi-isolant.

Un contact métallique **66** est placé sur la face avant de la couche n-GaN **62** et un autre contact métallique **67** est placé à l'extrémité de la colonne de p-GaN **64** correspondant à la zone de la rétine **68** à stimuler. Parce que d'une part la lumière **L** incidente doit pouvoir traverser le substrat p-GaN **61** pour atteindre la couche amplificatrice GaN **60**, et que d'autre part la lumière amplifiée LA doit pouvoir parvenir à la zone à stimuler **68**, le contact métallique **67** ne doit pas couvrir la totalité de la surface de l'extrémité de la colonne p-GaN **64**.

Les réflecteurs DBR **63** définissent une cavité optique dans laquelle la lumière bleue est amplifiée. Toute la lumière bleue est réfléchie sur le miroir constitué par le contact métallique **66** couvrant la surface de la face avant de la couche n-GaN **62**.

Après la réalisation d'une opération optogénétique, les cellules de la rétine vont être stimulées de façon sélective par la lumière bleue amplifiée **LA**. Un revêtement antireflet **69** est nécessaire à l'extrémité supérieure de la colonne p-GaN **64** afin d'éviter les réflexions parasites, et pour améliorer la qualité de la transmission optique.

La figure 7 illustre schématiquement un mode de réalisation d'un dispositif optoélectronique, comprenant au moins un amplificateur optique semiconducteur à base de GaN à cavité verticale, destiné à être utilisé dans une prothèse épirétinienne.

Une couche active GaN **70** amplificatrice à multiple puits quantiques InGaN/GaAsN ou InGaN/AlGaN est réalisée par épitaxie sur un substrat **71** de nitrure de gallium GaN dopé p. Une couche **72** de nitrure de gallium GaN dopée n est déposée au-dessus de la couche amplificatrice GaN **70**. Deux réflecteurs de Bragg distribués DBR **73** (pour « Distributed Bragg Reflector » en anglais) sont disposés respectivement de part et d'autre de la couche amplificatrice GaN **70**.

Par amincissement et polissage du substrat p-GaN **71**, on dégage une colonne de p-GaN **74** jusqu'au niveau de hauteur souhaité. La colonne de p-GaN **74** est recouverte d'une couche isolante **75** en matériau diélectrique ou en matériau semi-isolant.

Un contact métallique **76** est placé sur la face avant de la couche n-GaN **72** et un autre contact métallique **77** est placé à l'extrémité de la colonne de p-GaN **74** correspondant à la zone de la rétine **78** à stimuler. Parce que la lumière bleue **L** incidente doit traverser la couche n-GaN **72** pour atteindre la couche amplificatrice GaN **70**, le contact métallique **76** ne doit pas couvrir la totalité de la surface de la face avant de la couche n-GaN **72**. La lumière bleue LA une fois amplifiée doit pouvoir sortir de la colonne de p-GaN **74** afin d'aller stimuler les couches proches **78** de la rétine, où la thérapie optogénétique a été active, et le contact métallique **77** ne doit donc pas recouvrir la totalité de la surface de l'extrémité de la colonne de p-GaN **74**.

La lumière bleue est amplifiée dans la cavité optique définie par les deux réflecteurs de Bragg distribués **73**, positionnés de part et d'autre de la couche d'amplification GaN **70**. Après une opération optogénétique, les cellules de la rétine seront stimulées sélectivement par cette lumière bleue amplifiée **LA**. Un revêtement antireflet **79** est nécessaire à l'extrémité supérieure de la colonne p-GaN **74** afin d'éviter les réflexions parasites, et pour améliorer la qualité de la transmission optique.

Il peut être avantageux de combiner un dispositif optoélectronique destiné à une prothèse sous-rétinienne et un dispositif optoélectronique destiné à une prothèse épirétinienne, qu'ils aient ou non le même mode de fonctionnement. Par exemple on peut combiner une prothèse sous-rétinienne comprenant des amplificateurs optiques et une prothèse épirétinienne comprenant des photodiodes, notamment dans le cas où la thérapie optogénétique se révèle plus performante pour les cellules proches de la couche de cellules ganglionnaires que pour les cellules photoréceptrices comme les cônes. Ou inversement on peut combiner une prothèse épirétinienne comprenant des amplificateurs optiques et une prothèse sous-rétinienne comprenant des photodiodes. Il est également possible de combiner dans une même prothèse épirétinienne ou sous-rétinienne des photodiodes et des amplificateurs optiques. Ceci est réalisable par la mise en oeuvre de vias (trous métallisés) pour réaliser une polarisation directe et indirecte des composants optoélectroniques.

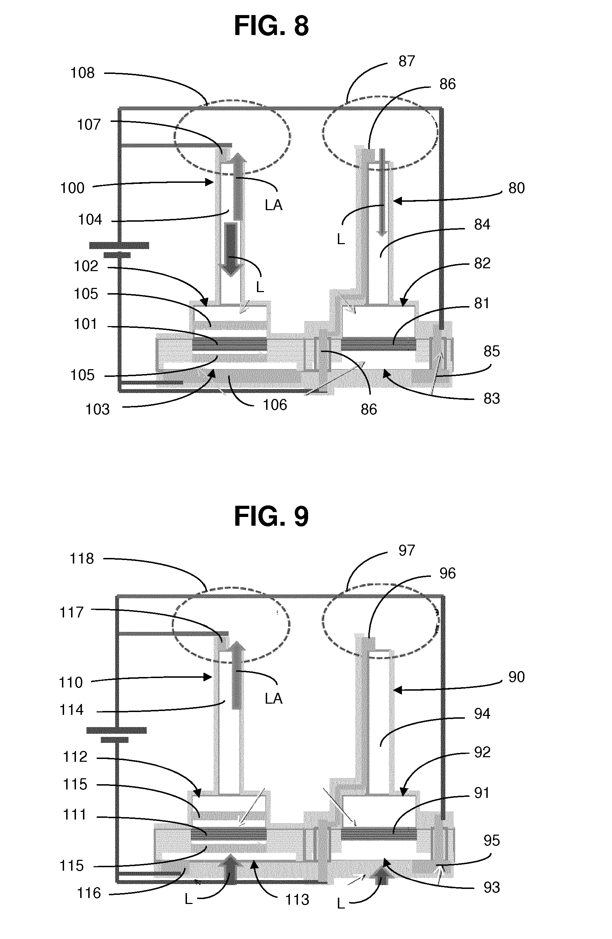

Dans le mode de réalisation illustré schématiquement sur la figure 8, un dispositif optoélectronique comprenant au moins une photodiode à base de GaN et au moins un amplificateur optique semiconducteur à base de GaN à cavité verticale combinés, est destiné à être utilisé dans une prothèse sous-rétinienne.

La photodiode **80,** analogue à celle de la figure 4, comprend une couche active GaN **81** absorbante à multiple puits quantiques InGaN/GaAsN ou InGaN/AlGaN déposée sur un substrat p-GaN **82** et surmontée d'une couche n-GaN **83** qui est découpée pour former une colonne de p-GaN **84.** Un contact métallique **85** est déposé sur la couche n-GaN **83** et un autre contact métallique **86** recouvre partiellement l'extrémité supérieure de la colonne de p-GaN **84** correspondant à la zone de la rétine **87** qui doit être stimulée.

L'amplificateur optique semiconducteur **100** à cavité verticale comprend une couche amplificatrice GaN **101** à multiple puits quantiques InGaN/GaAsN ou InGaN/AlGaN déposée sur un substrat p-GaN **102** et surmontée d'une couche n-GaN **103** qui est découpée pour former une colonne de p-GaN **104.** Deux réflecteurs de Bragg distribués DBR **105** sont disposés de part et d'autre de la couche amplificatrice GaN **101.** Un contact métallique **106** est déposé sur la couche n-GaN **103** et un autre contact métallique **107** recouvre partiellement l'extrémité supérieure de la colonne de p-GaN **102** correspondant à la zone de la rétine **108** qui doit être stimulée.

Il est possible d'adapter la structure à multiples puits quantiques de la photodiode et la structure à multiples puits quantiques de l'amplificateur à semiconducteur à cavité verticale avec réflecteurs de Bragg distribués, en faisant une épitaxie de jonction bout-à-bout.

On considérera maintenant la figure 9 qui illustre schématiquement un mode de réalisation d'un dispositif optoélectronique, comprenant au moins une photodiode à base de GaN et au moins un amplificateur optique semiconducteur à base de GaN à cavité verticale combinés, destiné à être utilisé dans une prothèse épirétinienne.

La photodiode **90,** analogue à celle de la figure 5, comprend une couche active GaN **91** absorbante à multiple puits quantiques InGaN/GaAsN ou InGaN/AlGaN déposée sur un substrat p-GaN **92** et surmontée d'une couche n-GaN **93** qui est découpée pour former une colonne de p-GaN **94.** Un contact métallique **95** est déposé sur la couche n-GaN **93** et un autre contact métallique **96** recouvre partiellement l'extrémité supérieure de la colonne de p-GaN **94** correspondant à la zone de la rétine **97** qui doit être stimulée.

L'amplificateur optique semiconducteur **110** à cavité verticale comprend une couche amplificatrice GaN **111** à multiple puits quantiques InGaN/GaAsN ou InGaN/AlGaN déposée sur un substrat p-GaN **112** et surmontée d'une couche n-GaN **113** qui est découpée pour former une colonne de p-GaN **114.** Deux réflecteurs de Bragg distribués DBR **115** sont disposés de part et d'autre de la couche amplificatrice GaN **111.** Un contact métallique **116** est déposé sur la couche n-GaN **113** et un autre contact métallique **117** recouvre partiellement l'extrémité supérieure de la colonne de p-GaN **112** correspondant à la zone de la rétine **118** qui doit être stimulée.

Ainsi le remplacement de la rétine par une prothèse comprenant un dispositif optoélectronique contenant des milliers, voire des millions, de composants optoélectroniques en matrice, comme illustré sur la figure 10, devient possible.

Un paramètre important est la distance entre deux composants optoélectroniques dans une matrice. Il est nécessaire d'avoir suffisamment d'espace libre entre les composants optoélectroniques pour que les cellules actives de la couche nucléaire interne CNI ou de la couche de cellules ganglionnaires CCG puissent continuer à fonctionner normalement. En particulier, il peut être intéressant de permettre l'introduction de tissus organiques entre les composants optoélectroniques individuels. Mais il est aussi nécessaire d'avoir un nombre suffisant de composants optoélectroniques (photodiodes ou amplificateurs optiques) pour assurer au patient une bonne définition de l'image.

Le fovéola a approximativement un diamètre d'environ 0,35 mm. L'espacement **E** entre deux dispositifs adjacents est donnée par la relation suivante, où n est le nombre de composants optoélectroniques de la matrice :

$$E\ (\mu m) = \pi\ (350/2)^2 \times 1/n$$

Dans le cas d'une matrice comprenant 2000 composants optoélectroniques, l'espacement **D** est d'environ 48 $\mu$m. La colonne de p-GaN doit avoir une hauteur **H** inférieure à 480 $\mu$m, sachant que l'épaisseur de la rétine est généralement inférieure à 0,5 mm. Dans un dispositif optoélectronique comportant des composants optoélectroniques dans lesquels la colonne de p-GaN a une dimension transversale **D** (largeur ou diamètre) d'environ 24 $\mu$m, il reste environ 24 $\mu$m disponibles pour permettre par exemple la présence des contacts métalliques et des liaisons électriques.

Bien entendu, la présente invention n'est pas limitée aux modes de réalisation décrits, mais elle est susceptible de nombreuses variantes accessibles à l'homme de l'art sans que l'on s'écarte de l'esprit de l'invention. En particulier, on pourra modifier la composition de la couche active pour tout semiconducteur III-V accordé sur le GaN et actif dans le domaine visible, c'est à dire ayant un pic de photoluminescence dans la zone bleu-vert-jaune.

**Revendications**

1. Dispositif optoélectronique comportant une matrice incluant au moins un composant optoélectronique comprenant une couche active de nitrure de gallium GaN comportant de multiples puits quantiques In-GaN/GaAsN (nitrure d'indium-gallium / nitrure d'arsenic-gallium) ou InGaN/AlGaN (nitrure d'indium-gallium / nitrure d'aluminium-gallium) sur un substrat de nitrure de gallium GaN dopé p et recouverte d'une couche de nitrure de gallium GaN dopé n.

2. Dispositif selon la revendication 1, dans lequel le substrat de nitrure de gallium GaN dopé p forme une colonne de p-GaN.

3. Dispositif selon la revendication 2, dans lequel la colonne de p-GaN est recouverte d'une couche isolante en matériau biocompatible choisi parmi le carbone, le diamant, le dioxyde de titane, la silice, le nitrure de silicium ou le nitrure de gallium.

4. Dispositif selon l'une des revendications 2 et 3, dans lequel le rapport entre la hauteur et la dimension transversale de la colonne de p-GaN est inférieur à 20.

5. Dispositif selon l'une des revendications 1 à 4, dans lequel le composant optoélectronique est une photodiode.

6. Dispositif selon l'une des revendications 1 à 4, dans lequel le composant optoélectronique est un amplificateur optique semiconducteur à cavité verticale.

7. Dispositif selon la revendication 6, dans lequel deux réflecteurs de Bragg distribués sont disposés respectivement de part et d'autre de la couche active GaN comportant de multiples puits quantiques de manière à définir une cavité optique.

8. Dispositif selon l'une des revendications précédentes, comportant une matrice comprenant au moins une photodiode et au moins un amplificateur optique semiconducteur à cavité verticale.

9. Dispositif selon l'une des revendications précédentes, comportant une matrice comprenant une pluralité de composants optoélectroniques.

10. Dispositif selon la revendication 9, dans lequel les composants optoélectroniques ont des hauteurs différentes.

11. Dispositif selon l'une des revendications 9 et 10, dans lequel les composants optoélectroniques sont espacés d'une distance E telle que $E = \pi (350/2)^2 \times 1/n$ où n est le nombre de composants optoélectroniques de la matrice.

12. Dispositif selon l'une des revendications 9 à 11, dans lequel la matrice de composants optoélectroniques est une matrice en trois dimensions de photodiodes et/ou d'amplificateurs optiques semiconducteurs à cavité verticale.

13. Prothèse épirétinienne comprenant un dispositif optoélectronique selon l'une des revendications 1 à 12.

14. Prothèse épirétinienne selon la revendication 13, comprenant une matrice de photodiodes.

15. Prothèse épirétinienne selon la revendication 13, comprenant une matrice d'amplificateurs optiques semiconducteurs à cavité verticale.

16. Prothèse épirétinienne selon la revendication 13, comprenant une matrice comportant des photodiodes et des amplificateurs optiques semiconducteurs à cavité verticale.

17. Prothèse sous-rétinienne comprenant un dispositif optoélectronique selon l'une des revendications 1 à 12.

18. Prothèse sous-rétinienne selon la revendication 17, comprenant une matrice de photodiodes.

19. Prothèse sous-rétinienne selon la revendication 17, comprenant une matrice d'amplificateurs optiques semiconducteurs à cavité verticale.

20. Prothèse sous-rétinienne selon la revendication 17, comprenant une matrice comportant des photodiodes et des amplificateurs optiques semiconducteurs à cavité verticale.

# FIG. 1

# FIG. 2

# FIG. 3

**(a)**         **(b)**         **(c)**

# FIG. 10

# FIG. 4

# FIG. 5

# FIG. 6

# FIG. 7

EP 3 062 352 A1

# FIG. 8

# FIG. 9

13

Europäisches Patentamt

European Patent Office

Office européen des brevets

# RAPPORT DE RECHERCHE EUROPEENNE

Numéro de la demande

EP 15 30 5288

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | QIN WANG ET AL: "Analysis and comparison of UV photodetectors based on wide bandgap semiconductors", PROC. SPIE 7602, GALLIUM NITRIDE MATERIALS AND DEVICES V, 11 mars 2010 (2010-03-11), pages 760225 1-10, XP040550549, DOI: 10.1117/12.849460 * abrégé; figures 1,2 * * pages 760225-1 - pages 760225-2 * ----- | 1-12 | INV. H01L31/0304 H01L31/0352 H01L27/146 H01L31/105 A61F2/14 A61N1/05 H01S5/50 |
| X | EP 0 896 405 A2 (CANON KK [JP]) 10 février 1999 (1999-02-10) * abrégé; figure 7 * * alinéas [0001], [0026], [0043] - [0049] * ----- | 1-4,6,7, 9-12 | |
| X | US 2008/144685 A1 (TANSU NELSON [US] ET AL) 19 juin 2008 (2008-06-19) * abrégé; figure 7 * * alinéas [0034] - [0038], [0074] - [0076] * ----- | 1-4,6,7, 9-12 | DOMAINES TECHNIQUES RECHERCHES (IPC) |
| A | PEREIRO J ET AL: "Design of InGaN based photodiodes by internal field engineering", ELECTRON DEVICES, 2007 SPANISH CONFERENCE ON, IEEE, PI, 2007, pages 296-299, XP031115317, ISBN: 978-1-4244-0868-9 * abrégé; figure 1 * * page 296 - page 297 * ----- -/-- | 1-6,10 | H01L A61F A61N H01S |

Le présent rapport a été établi pour toutes les revendications

| Lieu de la recherche | Date d'achèvement de la recherche | Examinateur |
|---|---|---|
| Berlin | 3 septembre 2015 | Sagol, Bülent Erol |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

..........................................................................

& : membre de la même famille, document correspondant

EPO FORM 1503 03.82 (P04C02)

Europäisches Patentamt
European Patent Office
Office européen des brevets

**RAPPORT DE RECHERCHE EUROPEENNE**

Numéro de la demande

EP 15 30 5288

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | LOUDIN JAMES ET AL: "Photovoltaic retinal prosthesis", OPHTHALMIC TECHNOLOGIES XXI, SPIE, 1000 20TH ST. BELLINGHAM WA 98225-6705 USA, vol. 7885, no. 1, 10 février 2011 (2011-02-10), pages 1-13, XP060005762, DOI: 10.1117/12.876560 [extrait le 2011-02-11] * le document en entier * | 1,3,5,6, 8-14, 16-18,20 | |
| A | PATRICK DEGENAAR ET AL: "Optobionic vision-a new genetically enhanced light on retinal prosthesis", JOURNAL OF NEURAL ENGINEERING, INSTITUTE OF PHYSICS PUBLISHING, BRISTOL, GB, vol. 6, no. 3, 20 mai 2009 (2009-05-20), page 35007, XP020162278, ISSN: 1741-2552 * le document en entier * | 1-4, 9-13,17 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 3 septembre 2015 | Sagol, Bülent Erol |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 15 30 5288

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-09-2015

| Document brevet cité au rapport de recherche | Date de publication | Membre(s) de la famille de brevet(s) | Date de publication |
|---|---|---|---|
| EP 0896405        A2 | 10-02-1999 | EP     0896405 A2<br>JP   H11154774 A<br>US     6261859 B1 | 10-02-1999<br>08-06-1999<br>17-07-2001 |
| US 2008144685    A1 | 19-06-2008 | AUCUN | |

Pour tout renseignement concernant cette annexe : voir Journal Officiel de l'Office européen des brevets, No.12/82

EPO FORM P0460